# EUROPEAN PATENT APPLICATION

(11) **EP 4 847 014 A1**
(43) Date of publication of application: **02.12.2026**
(21) Application number: 24920665.7
(22) Date of filing: 10.07.2024
(51) Int. Cl.: A61K 9/50

(54) **METHOD FOR LOADING USEFUL MATERIALS INTO CARRIER**

(30) Priority: 25.01.2024 KR 20240011347
(71) Applicant: STEMON Inc., Seongnam-si, Gyeonggi-do 13201 (KR)
(72) Inventor: KIM, Soon Hag, Seongnam-si, Gyeonggi-do 13589 (KR)
(74) Representative: Mewburn Ellis LLP
(86) International application number: PCT/KR2024/009826
(87) International publication number: WO 2025/159249

(57) **Abstract**

The present invention relates to a method for loading a useful substance into a carrier and is capable of maximizing the loading efficiency of a useful substance into a carrier such as an exosome by utilizing sonication. Accordingly, the present invention may be utilized in a technology for effectively delivering various useful substances, such as deoxyribonucleic acid, proteins, and nanomaterials, to target cells.

## Description

### TECHNICAL FIELD

The present invention relates to a method for loading a useful substance into a carrier.

### BACKGROUND ART

Many attempts have been made to load various substances, such as nucleic acids, proteins, and drugs, into carriers such as exosomes. Representative methods include incubation, electroporation, extrusion, freeze-thaw cycle, chimeric exosome method, endogenous loading, and sonication. According to investigations, in the case of the protein catalase, the loading efficiency through sonication is about 26 ± 1.2%, and it has been reported that substances can be loaded with a somewhat higher efficiency than by other methods such as extrusion (22 ± 3.1%), surfactant treatment (18.5 ± 1.3%), freeze-thaw treatment (14.7 ± 1.1%), and incubation (4.9 ± 0.5%). It has also been reported that loading of a chemotherapeutic agent (28.29 ± 1.38%) through sonication exhibits a higher efficiency than electroporation (5.30 ± 0.48%). Nanomaterials (gold nanomaterials) can also be loaded with high efficiency through sonication (19.3 ± 10%) (incubation: 13.7 ± 9.9%).

However, although the efficiency of loading useful substances into exosomes through sonication is about 19.3 to 28.3%, which is higher than that of other methods, it is still less than 30%, and thus there is a need to further improve the loading efficiency. Improving the loading efficiency has not only significant advantages in future processes and costs, but also a great advantage in maximizing the effect on recipient cells even when only a small amount is used. Accordingly, studies are being conducted to optimize a sonication loading method that can be utilized as a useful protein in various cosmetics, health functional foods, or medical devices, and to develop a method capable of maximizing the loading effect.

### DETAILED DESCRIPTION OF THE INVENTION

### TECHNICAL PROBLEM

A technical problem to be achieved by the present invention is to provide a method for loading a useful substance into a carrier.

The technical problem to be achieved by the present invention is not limited to the technical problem mentioned above, and other technical problems not mentioned herein will be clearly understood by those skilled in the art from the following description.

### TECHNICAL SOLUTION

In order to achieve the above technical problem, one embodiment of the present invention provides a method for loading a useful substance into a carrier, comprising: providing ultrasonic stimulation to each of a carrier comprising a phospholipid layer and a useful substance; and culturing a mixture of the ultrasonically stimulated carrier and useful substance for a predetermined period of time.

In an embodiment of the present invention, the carrier may be an exosome.

In an embodiment of the present invention, the carrier may be a human fibroblast-derived exosome (HDF-derived exosome).

In an embodiment of the present invention, the exosome may be prepared by a method comprising: providing ultrasonic stimulation to human fibroblast-derived cells and providing ultrasonic stimulation to a cell-free culture medium; culturing a mixture obtained by mixing the ultrasonically stimulated human fibroblast-derived cells and the ultrasonically stimulated culture medium for a predetermined period of time; and isolating exosomes from the mixture.

In an embodiment of the present invention, the useful substance may be a nucleic acid, protein, peptide, nanomaterial, plasmid DNA, low-molecular-weight compound, drug, hormone, enzyme, or neurotransmitter.

In an embodiment of the present invention, the providing ultrasonic stimulation to the carrier may be performed at an AMP intensity of 20% to 40% for 1 second to 10 seconds.

In an embodiment of the present invention, the providing ultrasonic stimulation to the useful substance may be performed at an AMP intensity of 15% to 30% for 0.1 second to 5 seconds.

In an embodiment of the present invention, when the useful substance is plasmid DNA, the ultrasonic stimulation may be performed at an AMP intensity of 15% to 25% for 0.1 second to 3 seconds.

In an embodiment of the present invention, when the useful substance is a protein, nucleic acid, or nanomaterial, the ultrasonic stimulation may be performed at an AMP intensity of 20% to 30% for 1 second to 5 seconds.

In an embodiment of the present invention, the culturing of the mixture may be performed at 30°C to 40°C for 30 minutes to 2 hours.

### ADVANTAGEOUS EFFECTS

The present invention relates to a method for loading a useful substance into a carrier, and is capable of maximizing the loading efficiency of a useful substance into a carrier such as an exosome by utilizing sonication. Therefore, the present invention may be utilized in a technology for effectively delivering various useful substances, such as deoxyribonucleic acid, proteins, and nanomaterials, to target cells.

The effects of the present invention are not limited to the effects described above, and should be understood to include all effects that can be inferred from the constitution of the invention described in the specification or claims of the present invention.

### DESCRIPTION OF THE DRAWINGS

FIG. 1 shows a comparison of loading efficiencies according to culture conditions of a mixture of sonicated exosomes and useful substances.
FIG. 2 shows the loading efficiency of a useful substance when sonication was performed once for 5 seconds at an AMP intensity of 30% under a culture condition of 37°C for 1 hour.
FIG. 3 shows the loading efficiency of a useful substance when sonication was performed once for 1 second at an AMP intensity of 20% under a culture condition of 4°C for 1 hour. The sonication conditions (useful substance: AMP 20%, 1 second, 1 pulse/exosome: AMP 30%, 5 seconds, 1 pulse), the number of exosome particles: 2×10⁸ particles/sample, and the loading amount of useful substance are shown in Table 1.
FIG. 4 shows the loading efficiency of a useful substance when sonication was performed once for 1 second at an AMP intensity of 20% under a culture condition of 37°C for 1 hour. The sonication conditions (useful substance: AMP 20%, 1 second, 1 pulse/exosome: AMP 30%, 5 seconds, 1 pulse), the number of exosome particles: 2×10⁸ particles/sample, and the loading amount of useful substance are shown in Table 1.
FIG. 5 shows a comparison of the loading efficiencies of a useful substance (Q-dot) according to whether sonication was performed. Number of exosome particles: 2×10⁸ particles/sample; Q-dot 705 loading amount: 1 nmole (0.01 µL); sonication condition: AMP 30%, 5 seconds, 1 pulse; culture condition: 37°C, 1 hour; DiD staining: 37°C, 30 minutes.
FIGS. 6 and 7 show whether useful substances (IgG and Cy3-DNA) were damaged according to sonication conditions.
FIG. 8 shows whether plasmid DNA was loaded into exosomes.
FIG. 9 shows whether plasmid DNA was damaged according to sonication conditions.
FIGS. 10 to 13 show whether useful substances loaded into exosomes were delivered to cells.

### BEST MODE FOR CARRYING OUT THE INVENTION

Hereinafter, the present invention will be described in detail.

The present invention relates to a method for loading a useful substance into a carrier.

The present invention comprises providing ultrasonic stimulation to each of a carrier comprising a phospholipid layer and a useful substance; and culturing a mixture of the ultrasonically stimulated carrier and useful substance for a predetermined period of time.

The carrier comprises a phospholipid layer, and the type and form thereof are not particularly limited as long as a core is formed therein by the phospholipid layer and a useful substance can be carried (loaded) in the core. For example, the carrier may be an exosome, and more specifically, may be a human fibroblast-derived exosome (HDF-derived exosome).

The exosome is isolated from cells and may be isolated using known means according to known methods and conditions. For example, the exosome may be prepared by a method comprising: providing ultrasonic stimulation to human fibroblast-derived cells and providing ultrasonic stimulation to a cell-free culture medium; culturing a mixture obtained by mixing the ultrasonically stimulated human fibroblast-derived cells and the ultrasonically stimulated culture medium for a predetermined period of time; and isolating exosomes from the mixture.

The providing ultrasonic stimulation to the HDF-derived cells may be performed by directly sonicating the cells or under conditions in which only a minimum amount of an initial culture medium sufficient to barely cover the cells is present. The initial culture medium may be a general medium used to maintain the cells in a healthy state and may be, for example, a DMEM medium containing antibiotics and serum.

The cell-free culture medium is not particularly limited in type and may be, for example, any one of an embryonic stem cell medium, neural stem cell medium, cardiac stem cell medium, dermal papilla cell medium, mesenchymal stem cell medium, osteogenic medium, myogenic medium, hematopoietic stem cell medium, neuron medium, astrocyte medium, oligodendrocyte medium, hepatocyte medium, adipocyte medium, muscle cell medium, vascular endothelial cell medium, pancreatic beta-cell medium, and cardiomyocyte medium.

The ultrasonic stimulation provided to the cells may be performed, for example, at 10 to 30 kHz and 0.5 to 3 W/cm² for 1 to 10 seconds, and preferably at 15 to 25 kHz and 0.5 to 1.5 W/cm² for 3 to 7 seconds.

The ultrasonic stimulation provided to the cell-free culture medium may be performed at 10 to 30 kHz and 1 to 20 W/cm² for 1 minute to 20 minutes, and preferably at 15 to 25 kHz and 1 to 10 W/cm² for 5 minutes to 15 minutes.

The culturing of the mixture obtained by mixing the ultrasonically stimulated human fibroblast-derived cells and the ultrasonically stimulated culture medium may be performed using known means according to known methods and conditions. For example, the culturing may be performed for 1 day to 10 days, 1 day to 6 days, or 1 day to 2 days. Since exosomes are secreted in the greatest amount on the first day after sonication and the amount of secretion decreases over time, the culture time may be appropriately selected within the above range.

The isolating exosomes from the mixture may be performed using known means according to known methods and conditions. For example, the isolating may comprise: centrifuging the cultured mixture to obtain a supernatant; filtering the supernatant through a filter to obtain a filtrate; and concentrating the filtrate.

The centrifugation is performed to remove cell debris and dead cells and may preferably be performed at 1,000 g to 5,000 g for 10 minutes to 60 minutes.

The filtering the supernatant through a filter is performed to further remove cell debris and retain only particles below a specific size, and the filter used herein may preferably be a syringe filter.

The concentrating the filtrate may preferably be performed using a centrifugal filter. By using a centrifugal filter, the filtrate may be concentrated while simultaneously removing particles below a specific size.

The isolating exosomes may further comprise, as necessary, storing the supernatant for 7 days to 3 months at 4°C or below before filtering the supernatant through a filter. The storage may preferably be performed within 7 days at 4°C or below, more preferably within 1 month at -20°C or below, and most preferably within 3 months at -80°C or below.

The useful substance is a physiologically active substance and may include any substance that enhances or suppresses a biological function. For example, the useful substance may include a nucleic acid, protein, peptide, nanomaterial, plasmid DNA, low-molecular-weight compound, drug, hormone, enzyme, or neurotransmitter.

The providing ultrasonic stimulation to the carrier may be performed, for example, at an AMP intensity of 20% to 40% for 1 second to 10 seconds. Preferably, the ultrasonic stimulation may be performed at an AMP intensity of 25% to 35% for 3 seconds to 7 seconds.

The providing ultrasonic stimulation to the useful substance may be performed, for example, at an AMP intensity of 15% to 30% for 0.1 second to 5 seconds or at an AMP intensity of 20% to 30% for 1 second to 5 seconds.

More specifically, when the useful substance is plasmid DNA, the ultrasonic stimulation may be performed at an AMP intensity of 15% to 25% for 0.1 second to 3 seconds. Preferably, the ultrasonic stimulation may be performed at an AMP intensity of 18% to 22% for 0.5 second to 2 seconds. In addition, when the useful substance is a protein, nucleic acid, or nanomaterial, the ultrasonic stimulation may be performed at an AMP intensity of 20% to 30% for 1 second to 5 seconds.

Although the ultrasonic stimulation conditions are not limited to the above ranges, when performed within the above ranges, the degree of damage to the useful substance may be minimized and the loading efficiency may be maximized.

The culturing of the mixture of the ultrasonically stimulated carrier and useful substance may be performed using known means according to known methods and conditions. For example, the culturing may be performed at 30°C to 40°C for 30 minutes to 2 hours. Preferably, the culturing may be performed at 35°C to 40°C for 50 minutes to 70 minutes.

Hereinafter, the present invention will be described in detail with reference to Examples in order to specifically illustrate the present invention.

### EXAMPLES

### Example 1. Preparation of Human Fibroblast-Derived Exosomes (HDFs)

Ultrasonic stimulation at 20 kHz and 1.0 W/cm² was directly applied for 5 seconds to 1×10⁶ human dermal fibroblasts (HDFs) using UltraRepro 1001 (STEMON Inc., Seoul, Republic of Korea) (hereinafter, the ultrasonically stimulated HDFs are referred to as "UHDFs"). 2×10⁵ UHDFs were cultured for one day in a 35-mm Petri dish together with an ultrasonically treated embryonic stem cell culture medium (20 kHz, 5.0 W/cm², 10 minutes) (DMEM/F12, 15% FBS, 2 mM GlutaMAX, 0.1% NEAA, 0.1% penicillin/streptomycin, 0.1 mM β-mercaptoethanol, and 1000 unit/ml leukemia inhibitory factor (LIF)). Exosomes were isolated from the culture medium in which the ultrasonically treated HDFs (UHDFs) had been cultured as follows. The culture medium was centrifuged at 3,000 × g for 20 minutes to remove cell debris and dead cells, and the supernatant was passed through a 0.22-µm filter (Minisart^{®} Syringe Filter, Sartorius, Goettingen, Germany). The resulting medium was loaded into an Amicon^{®} Ultra-15 100,000 kDa device (Millipore, Billerica, MA, USA) and centrifuged at 14,000 × g for 20 minutes to concentrate the exosomes.

### Example 2. Loading of Useful Substances into Exosomes

The exosomes prepared in Example 1 were dispensed into 1.5-ml tubes in required amounts, and DPBS was added thereto so that the final volume became 100 µL.

Useful substances to be loaded were dispensed into 1.5-ml tubes according to Table 1, and DPBS was added thereto so that the final volume became 100 µL.

**[Table 1]**

| **No .** | **Useful substance (loading substance)** | **Loading amount** | **Volume** |
|---|---|---|---|
| 1 | Deoxyribonucleotide (DNA) (Cy3-labeled DNA) | 20 ng | 100 µL (1 × 10¹¹ fluorescence particles) |
| 2 | Protein (IgG-488) | 0.1 µg/ml | 0.01 µL |
| 3 | Nanomaterial (Q-dot 705) | 1 nmole | 0.01 µL |
| 4 | Plasmid DNA | 1 µg | Variable depending on the isolated concentration of plasmid DNA |

The exosomes were subjected to sonication once for 5 seconds at an AMP intensity of 30%, and the useful substance to be loaded was subjected to sonication either once for 5 seconds at an AMP intensity of 30% or once for 1 second at an AMP intensity of 20%. The sonicated exosomes and useful substance were thoroughly mixed and then cultured at 37°C or 4°C for 30 minutes or 2 hours.

### Experimental Example 1. Comparison of Loading Efficiencies According to Culture Conditions of a Mixture of Sonicated Exosomes and Useful Substances

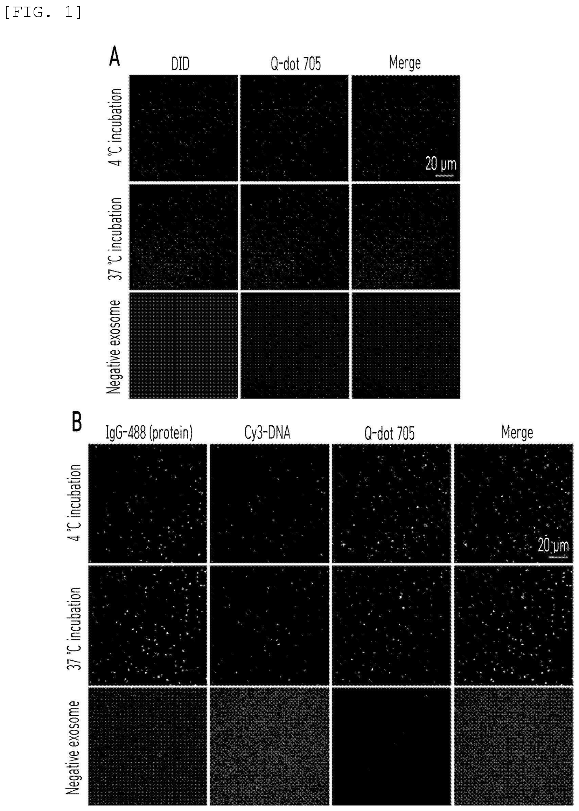

Culturing at 37°C was more effective in increasing the loading efficiency than culturing at 4°C, and under culture conditions of 30 minutes to 2 hours, the loading efficiency tended to increase as the culture time increased. However, for production efficiency, the culture time was determined to be 1 hour (FIG. 1).

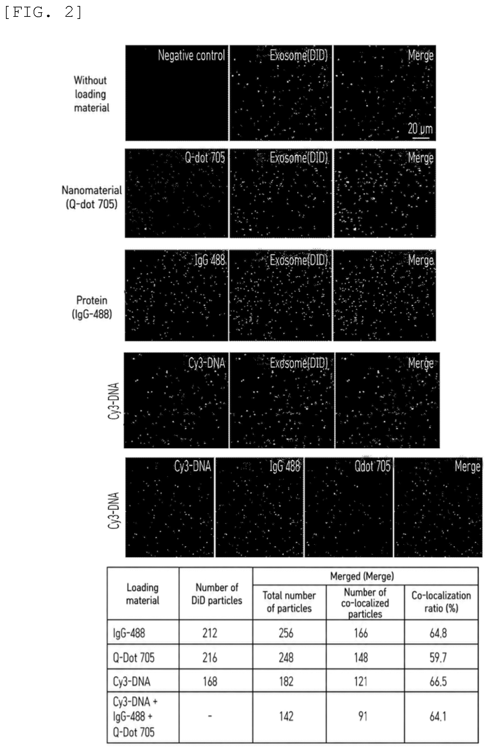

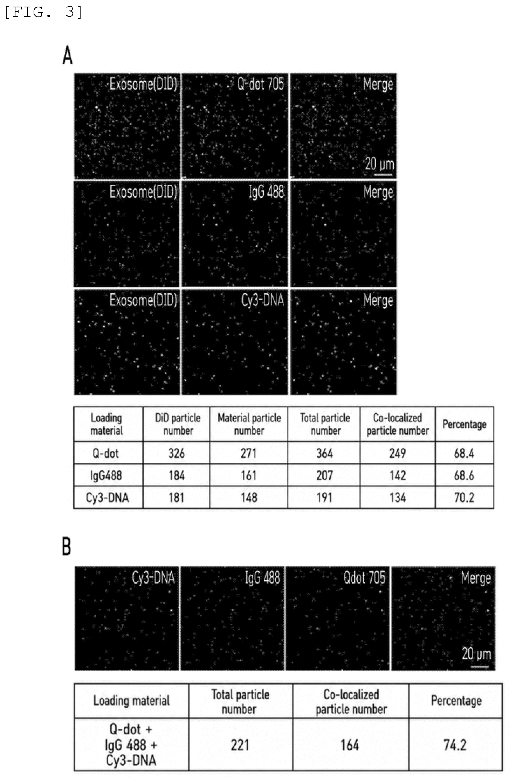

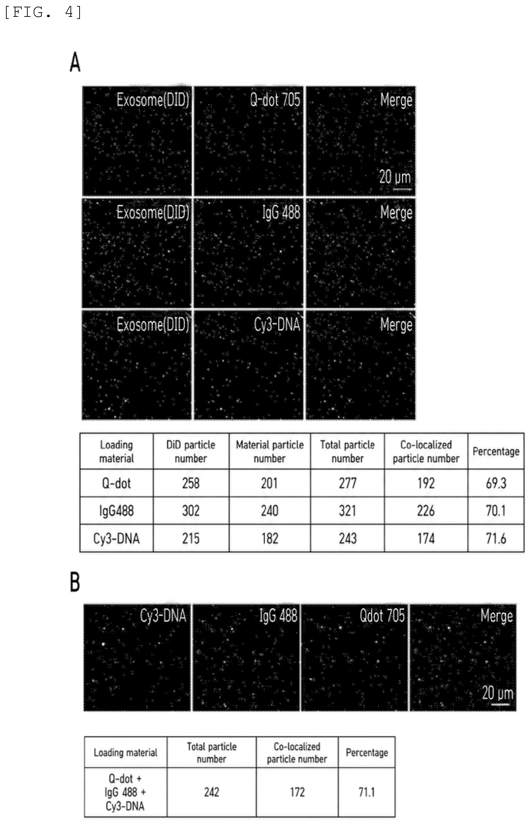

In addition, when sonication was performed once for 5 seconds at an AMP intensity of 30%, a loading efficiency of about 60% was observed (FIG. 2), whereas when sonication was performed once for 1 second at an AMP intensity of 20%, a loading efficiency of about 70% was observed (FIGS. 3 and 4).

### Experimental Example 2. Comparison of Loading Efficiencies According to Sonication Targets

A comparison was made among cases in which only the useful substance was sonicated, only the exosomes were sonicated, and both the useful substance and the exosomes were individually sonicated. The sonication was performed once for 5 seconds at an AMP intensity of 30%.

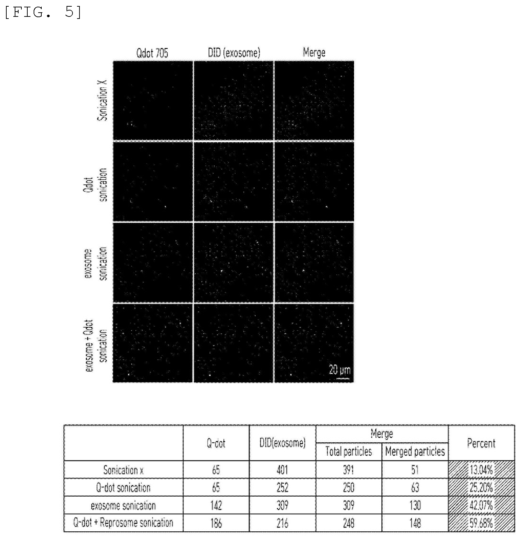

As a result, the loading efficiency was 13.04% when no sonication was performed, 25.2% when only the useful substance (Q-dot) was sonicated, 42.07% when only the exosomes were sonicated, and 59.68% when both the useful substance and the exosomes were individually sonicated. Accordingly, it was demonstrated that the loading efficiency of the substance was highest when the sonication technology of the present invention was applied (FIG. 5).

### Experimental Example 3. Confirmation of Damage to Useful Substances Caused by Sonication

In order to determine whether sonication causes damage to useful substances, useful substances (IgG and Cy3-DNA) were subjected either to sonication once for 5 seconds at AMP 30% or to sonication once for 1 second at AMP 20%, and then analyzed by SDS-PAGE (with immunoblot) using a 10% acrylamide gel and by 2% agarose gel electrophoresis.

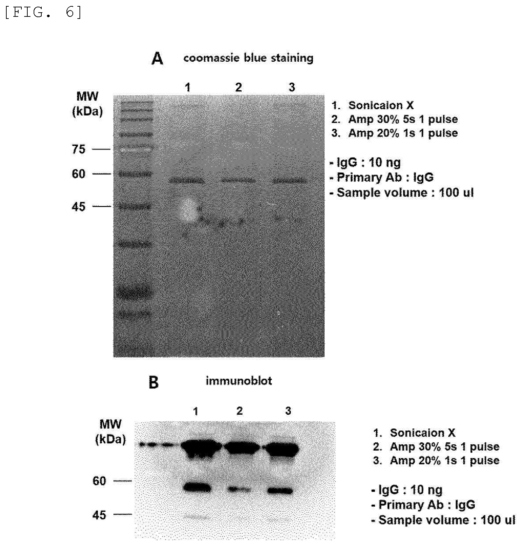

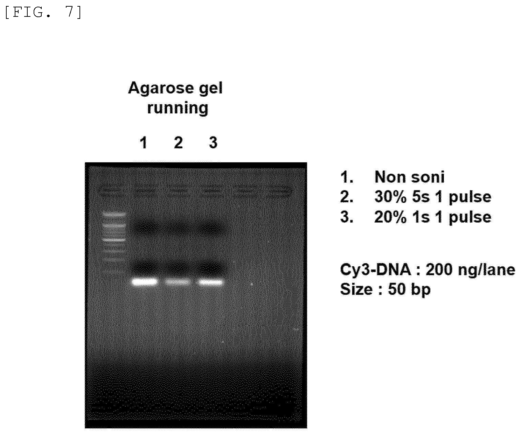

It was confirmed that when sonication was performed once for 5 seconds at AMP 30%, damage to the useful substances was induced, and the degree of damage was approximately 50% compared to the case where no sonication was performed. On the other hand, when sonication was performed once for 1 second at AMP 20%, only about 25% damage was induced (FIGS. 6 and 7). Accordingly, because sonication causes damage to useful substances, it is important to perform loading under appropriate sonication conditions, and it was confirmed that the sonication technology of the present invention can reduce damage to substances while increasing loading efficiency.

### Experimental Example 4. Plasmid DNA Loading Experiment

After loading plasmid DNA into exosomes, an experiment was conducted to determine whether the material loaded into the exosomes could be delivered to cells and properly perform its function by treating cells with the exosomes.

### (1) Confirmation of Whether Plasmid DNA Was Loaded into Exosomes

In order to determine whether loading of plasmid DNA into exosomes could be induced through sonication, sonication at AMP 30% for 5 seconds and one pulse was applied to: 1) exosomes only; 2) plasmid DNA only; or 3) both exosomes and plasmid DNA (negative control: no sonication). Thereafter, plasmid DNA present inside the exosomes was purified using a mini prep kit, and PCR was performed using the purified plasmid DNA.

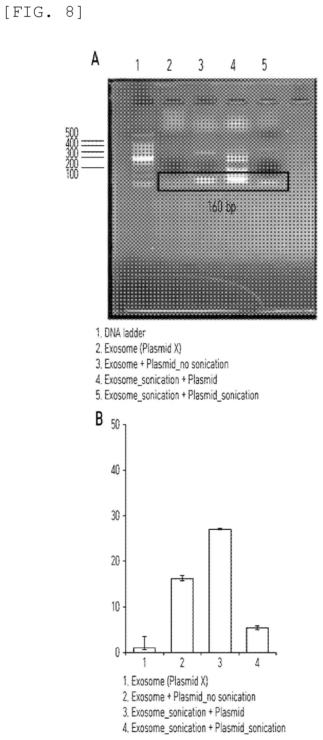

As a result, it was confirmed that a portion of the plasmid DNA was loaded into exosomes even without sonication. However, when sonication was applied only to the exosomes (loading through sonication), a larger amount of plasmid DNA was loaded. When sonication was applied to both the exosomes and plasmid DNA, the loading efficiency appeared to decrease. This was presumed to be due to damage to the plasmid DNA caused by sonication (FIG. 8).

Accordingly, an additional experiment for confirming plasmid DNA damage caused by sonication was conducted as described below.

### (2) Confirmation of Plasmid DNA Damage Caused by Sonication

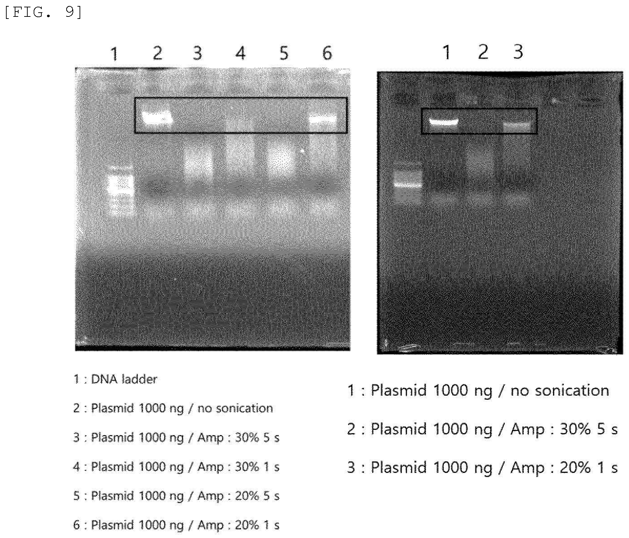

In order to confirm damage to plasmid DNA caused by sonication as in the previous experiment, plasmid DNA was subjected to sonication once for 5 seconds at AMP 30% or once for 1 second at AMP 20%, followed by analysis through 1% agarose gel electrophoresis (FIG. 9). As with the other useful substances described above, greater damage occurred under the condition of sonication once for 5 seconds at AMP 30% (approximately 90%), whereas a tendency toward reduced damage was observed under the condition of sonication once for 1 second at AMP 20% (approximately 50%).

Taken together with the foregoing experiments, the loading efficiencies and degrees of damage according to sonication conditions are shown in Table 2 below.

That is, when the loading efficiency was increased compared with approximately 20% in the conventional technology and the degree of damage to the useful substance was approximately 50%, the conditions were determined to be suitable for loading useful substances into exosomes.

**[Table 2]**

| | **AMP 30%, 5 s, 1 pulse** | **AMP 20%, 1 s, 1 pulse** | **Suitable condition** |
|---|---|---|---|
| Q-dot | Loading efficiency: 59.7% | Loading efficiency: 69.3% | Both |
| | Damage: - | Damage: - | |
| Protein | Loading efficiency: 64.8% | Loading efficiency: 70.1% | Both |
| | Damage: about 50% | Damage: about 25% | |
| Cy3 DNA | Loading efficiency: | Loading efficiency: | Both |
| | 66.5% | 71.6% | |
| | Damage: about 50% | Damage: about 25% | |
| Plasmid DNA | Loading efficiency: - | Loading efficiency: - | 20%, 1 s, 1 pulse |
| | Damage: about 90% | Damage: about 50% | |

(3) Cell Delivery of Useful Substances Loaded into Exosomes : Based on the foregoing experiment confirming the degree of damage, Q-dot, IgG488, or plasmid DNA was loaded into exosomes using a method in which sonication was performed once for 1 second at AMP 20%.

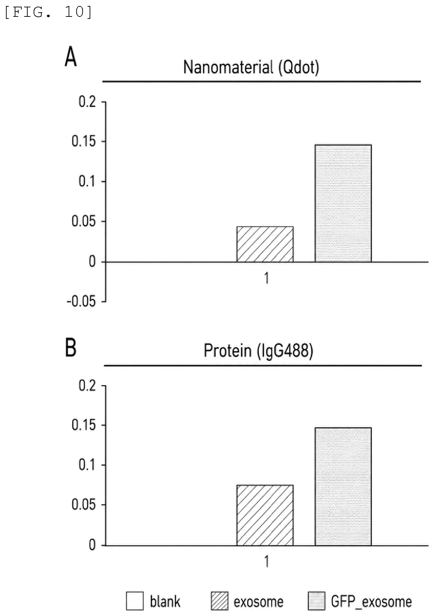

Thereafter, HDF cells were seeded into a 24-well cell culture plate at 1×10⁵ cells/well, and after 16 hours, exosomes loaded with Q-dot or IgG488 were added to the cells. Twenty-four hours later, fluorescence analysis was performed to determine whether the substances had been delivered to the cells. As a result, when exosomes loaded with useful substances were added to the cells, fluorescence measurements increased compared with untreated HDF cells, thereby confirming that exosomes loaded with useful substances were delivered to cells and could deliver the substances into the cells (FIG. 10).

To determine whether plasmid DNA loaded into exosomes could be delivered to cells and perform its function, plasmid DNA-loaded exosomes were prepared by applying sonication to 1×10⁹ particles of exosomes at AMP 30% for one pulse and to plasmid DNA at AMP 20% for 1 second and one pulse. The plasmid DNA-loaded exosomes were then added to cells, and intracellular delivery was directly and indirectly confirmed through antibiotic resistance, luciferase activity, and PCR, which are characteristics encoded by the plasmid.

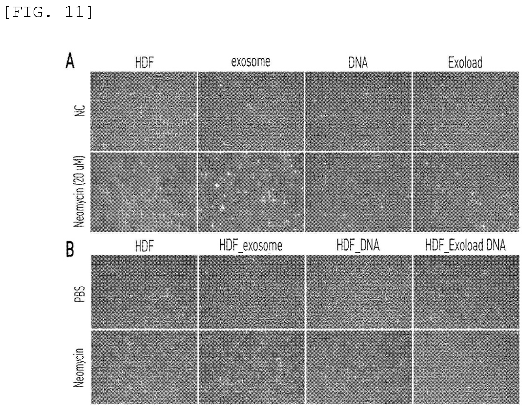

For treatment with exosomes loaded with plasmid DNA, cells were seeded into a 24-well cell culture plate at 1×10⁵ cells/well. Twenty-four hours after treatment with the plasmid DNA-loaded exosomes, an antibiotic was added, and resistance to the antibiotic was evaluated 24 hours thereafter. As a result, when neomycin was added to cells not loaded with plasmid DNA (HDF, Reprosome, DNA only), cell death was observed. In contrast, when cells were treated with exosomes loaded with plasmid DNA, cell death caused by neomycin was reduced (FIG. 11).

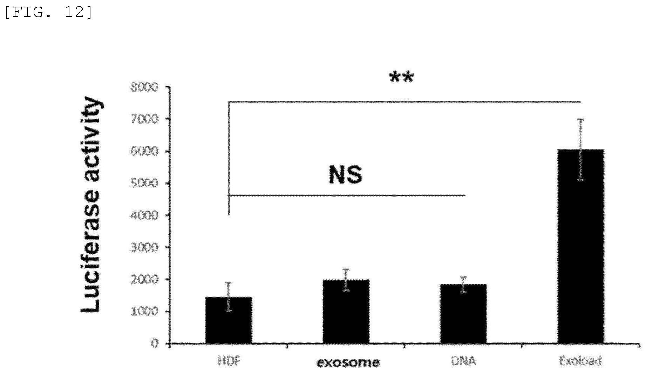

For the luciferase assay, HDF cells were seeded into a 24-well cell culture plate at 1×10⁵ cells/well, and after 16 hours, exosomes loaded with plasmid DNA were added to the cells. Thereafter, a luciferase assay was performed after 24 hours. As a result, increased luciferase activity was observed only in cells treated with exosomes loaded with plasmid DNA (FIG. 12).

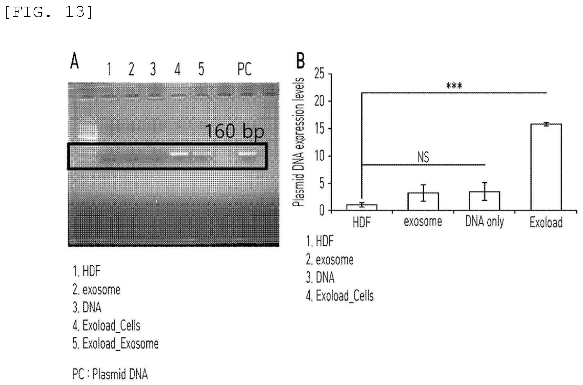

Finally, in order to confirm intracellular delivery of a substance (plasmid DNA delivery) through exosomes by synthesizing cDNA from intracellular mRNA and performing PCR, HDF cells were seeded into a 24-well cell culture plate at 1×10⁵ cells/well. Sixteen hours later, exosomes loaded with plasmid DNA were added to the cells, and after an additional 24 hours, luciferase RNA was purified, cDNA was synthesized, and PCR was performed (FIG. 13). As a result, a PCR band was detected when cells were treated with exosomes loaded with plasmid DNA (lane 4), and amplification was also confirmed by qPCR (lane 1: only cell; lane 2: treatment of exosome; lane 3: treatment of plasmid DNA only; lane 5: plasmid DNA purified from exosome; PC: positive control, original plasmid DNA).

## Claims

1. A method for loading a useful substance into a carrier, comprising:
providing ultrasonic stimulation to each of a carrier comprising a phospholipid layer and a useful substance; and
culturing a mixture of the ultrasonically stimulated carrier and useful substance for a predetermined period of time.

2. The method for loading a useful substance into a carrier according to claim 1, wherein the carrier is an exosome.

3. The method for loading a useful substance into a carrier according to claim 1, wherein the carrier is a human fibroblast-derived exosome (HDF-derived exosome).

4. The method for loading a useful substance into a carrier according to claim 3, wherein the exosome is prepared by a method comprising:
providing ultrasonic stimulation to human fibroblast-derived cells and providing ultrasonic stimulation to a cell-free culture medium;
culturing a mixture obtained by mixing the ultrasonically stimulated human fibroblast-derived cells and the ultrasonically stimulated culture medium for a predetermined period of time; and
isolating exosomes from the mixture.

5. The method for loading a useful substance into a carrier according to claim 1, wherein the useful substance is a nucleic acid, protein, peptide, nanomaterial, plasmid DNA, low-molecular-weight compound, drug, hormone, enzyme, or neurotransmitter.

6. The method for loading a useful substance into a carrier according to claim 1, wherein the providing ultrasonic stimulation to the carrier is performed at an AMP intensity of 20% to 40% for 1 second to 10 seconds.

7. The method for loading a useful substance into a carrier according to claim 1, wherein the providing ultrasonic stimulation to the useful substance is performed at an AMP intensity of 15% to 30% for 0.1 second to 5 seconds.

8. The method for loading a useful substance into a carrier according to claim 7, wherein, when the useful substance is plasmid DNA, the ultrasonic stimulation is performed at an AMP intensity of 15% to 25% for 0.1 second to 3 seconds.

9. The method for loading a useful substance into a carrier according to claim 7, wherein, when the useful substance is a protein, nucleic acid, or nanomaterial, the ultrasonic stimulation is performed at an AMP intensity of 20% to 30% for 1 second to 5 seconds.

10. The method for loading a useful substance into a carrier according to claim 1, wherein the culturing of the mixture is performed at 30°C to 40°C for 30 minutes to 2 hours.
